(19) **Europäisches Patentamt / European Patent Office / Office européen des brevets**

(11) **EP 4 764 452 A1**

(12) **EUROPEAN PATENT APPLICATION**
published in accordance with Art. 153(4) EPC

(43) Date of publication:
**24.06.2026 Bulletin 2026/26**

(21) Application number: **24872987.3**

(22) Date of filing: **26.09.2024**

(51) International Patent Classification (IPC):
**G01N 3/303** (2006.01)  **G01N 33/44** (2006.01)
**G06N 20/00** (2019.01)

(52) Cooperative Patent Classification (CPC):
**G01N 3/303; G01N 33/44; G06N 20/00**

(86) International application number:
**PCT/KR2024/014627**

(87) International publication number:
**WO 2025/071279 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.09.2023 KR 20230129784**
**25.09.2024 KR 20240130233**

(71) Applicant: **LG Chem, Ltd.**
**Seoul 07336 (KR)**

(72) Inventors:
• **MOON, Sung Nam**
**Daejeon 34122 (KR)**
• **JO, Seongin**
**Daejeon 34122 (KR)**

• **IM, Damhyeok**
**Daejeon 34122 (KR)**
• **JEON, Yeojin**
**Daejeon 34122 (KR)**
• **NA, Young Hoon**
**Daejeon 34122 (KR)**
• **LEE, Minkyoung**
**Daejeon 34122 (KR)**
• **HWANG, Seokyung**
**Daejeon 34122 (KR)**
• **JUNG, Jinmi**
**Daejeon 34122 (KR)**

(74) Representative: **Hoffmann Eitle**
**Patent- und Rechtsanwälte PartmbB**
**Arabellastraße 30**
**81925 München (DE)**

(54) **APPARATUS AND METHOD FOR DESIGNING MULTILAYER FILM**

(57) An apparatus and method for designing a multilayer film is disclosed. An apparatus for designing a multilayer film may perform: modeling a lamination structure of a multilayer film to be designed, collecting stress-strain data with respect to the single-layer film forming the lamination structure, reading a value pre-stored in a storage space accessible by an apparatus for designing a multilayer film, and obtaining a feature setting mode for designating different feature setting manners depending on the read value, calculating a strain energy from a plurality of physical indicators selected from the stress-strain data, depending on the feature setting mode, and setting the strain energy as the feature, selecting at least one among a plurality of supervised learning models capable of a regression analysis as a machine learning model, predicting the dart impact strength of the multilayer film by using the machine learning model learned by taking the feature as an independent variable, and a dart impact strength of the multilayer film as a target variable, and generating design data for the multilayer film, by combining predicted values for other properties and a predicted value of the dart impact strength, so as to satisfy the design requirements of the multilayer film.

【Figure 1】

## Description

### CROSS-REFERENCE TO RELATED APPLICATION

This application claims priority to and the benefit of Korean Patent Application No. 10-2023-0129784 filed with the Korean Intellectual Property Office on September 26, 2023, and Korean Patent Application No. 10-2024-0130233 filed with the Korean Intellectual Property Office on September 25, 2024, the entire contents of which are incorporated herein by reference.

### [Technical Field]

The present disclosure relates to an apparatus and method for designing a multilayer film.

### [Background Art]

The polymer film is a thin and flat structure made of polymer material. Here, polymer may have a molecule structure in the form of a long chain in which repeating units called monomers are connected. The thickness of the polymer film can be set to a variety of units, down to millimeters (mm). The polymer film can have various advantages, including flexibility, light weight, cost effectiveness, and processability. Specifically, the polymer film can be manufactured into various shapes and sizes due to their excellent flexibility, making them suitable for various product designs. In addition, the lightweight nature of polymer films makes them suitable for products requiring portability, thereby reducing transportation and handling costs. In addition, it is cost-effective because it can be manufactured with relatively inexpensive raw materials and can be mass-produced. In addition, since polymer films can be applied to various manufacturing and processing technologies such as thermal processing, extrusion, and bonding, the shape, size, and thickness of the product can be easily adjusted. In addition, polymer films have high durability and strength, where some have excellent optical properties, are waterproof, and can be breathable if required.

The multilayer film may be a film composed of two or more layers of polymers or other materials. This multilayer structure can improve the performance of the overall film by combining the unique properties of each layer. For example, one layer may be used to increase durability and strength, another layer may provide waterproofing or moisture resistance, and yet another layer may provide resistance to certain chemicals. In this way, by combining layers with different properties, multilayer films can adapt to a wider range of applications and specific requirements than single-layer films.

### [Disclosure]

### [Technical Problem]

An object to be solved is to provide an apparatus and method for designing a multilayer film capable of predicting the dart impact strength of the multilayer film, and designing the multilayer film according to given design requirements with only information on the single-layer film constituting the multilayer film when designing a multilayer film.

### [Technical Solution]

An apparatus for designing a multilayer film according to an embodiment is for designing the multilayer film based on a physical property of a multilayer film predicted from only information on a single-layer film, by executing a program code loaded on one or more memory devices through one or more processors, where the program code is configured, when executed, to perform modeling a lamination structure of a multilayer film to be designed, collecting stress-strain data with respect to the single-layer film forming the lamination structure, reading a value pre-stored in a storage space accessible by an apparatus for designing a multilayer film, and obtaining a feature setting mode for designating different feature setting manners depending on the read value, calculating a strain energy from a plurality of physical indicators selected from the stress-strain data, depending on the feature setting mode, and setting the strain energy as the feature, selecting at least one among a plurality of supervised learning models capable of a regression analysis as a machine learning model, predicting the dart impact strength of the multilayer film by using the machine learning model learned by taking the feature as an independent variable, and a dart impact strength of the multilayer film as a target variable, and generating design data for the multilayer film, by combining a predicted value for another property and a predicted value of the dart impact strength, so as to satisfy the design requirements of the multilayer film.

In some embodiments, when the feature setting mode may include a first feature setting mode, the setting as the feature may include setting, as the feature, at least some of yield stress, yield strain, necking stress, necking strain, breaking stress, and breaking strain, a strain energy calculated from at least some of the yield stress, the yield strain, the necking stress, the necking strain, the breaking stress, and the breaking strain, and a thickness of the single-layer film.

In some embodiments, the selecting as the machine learning model may include selecting one of a partial least squares (PLS) model, a Lasso regression model, and a random forest model as the machine learning model, and where the machine learning model selected from among the PLS model, the Lasso regression model, and the random forest model is learned to predict the dart impact strength of the multilayer film, by receiving a vector generated from at least some of the yield stress, the yield strain, the necking stress, the necking strain, the breaking stress, and the breaking strain, a vector generated from the strain energy calculated from at least some of the yield stress, the yield strain, the necking stress, the necking strain, the breaking stress, and the breaking strain, and a vector generated from the thickness of the single-layer film.

In some embodiments, when the feature setting mode may include a second feature setting mode, the setting as the feature may include setting, as the feature, yield stress, yield strain, necking stress, necking strain, breaking stress, and breaking strain, a strain energy calculated from the yield stress, the yield strain, the necking stress, the necking strain, the breaking stress, and the breaking strain, and a thickness of the single-layer film.

In some embodiments, the selecting as the machine learning model may include selecting one of a partial least squares (PLS) model, a Lasso regression model, and a random forest model as the machine learning model, and where the machine learning model selected from among the PLS model, the Lasso regression model, and the random forest model are learned to predict the dart impact strength of the multilayer film, by receiving a vector generated from the yield stress, the yield strain, the necking stress, the necking strain, the breaking stress, and the breaking strain, a vector generated from the strain energy calculated from the yield stress, the yield strain, the necking stress, the necking strain, the breaking stress, and the breaking strain, and a vector generated from the thickness of the single-layer film.

In some embodiments, the strain energy may include a first strain energy ($W_{yield}$), and where the setting as the feature may include calculating the first strain energy ($W_{yield}$) according to the following equation and setting the calculated first strain energy ($W_{yield}$) as the feature,

$$W_{yield} = a\pi d^2 t \sigma_y (\varepsilon_y) \left[ \left( \frac{\sigma_{br}}{\sigma_y} \right)^2 - 1 \right]$$

where, $\sigma_y$ denotes the yield stress, $\varepsilon_y$ denotes the yield strain, $\sigma_{br}$ denotes the breaking stress, d denotes a dart contact diameter, t denotes a thickness of a measurement sample, and a denotes an experimentally determined constant.

In some embodiments, the strain energy may include a second strain energy ($W_{neck}$), and where setting as the feature may include calculating the second strain energy ($W_{neck}$) according to the following equation and set the calculated second strain energy ($W_{neck}$) as the feature,

$$W_{neck} = b\pi d^2 t \sigma_n (\varepsilon_n - \varepsilon_y) \left[ \left( \frac{\sigma_{br}}{\sigma_y} \right)^2 - 1 \right]$$

where, $\sigma_n$ denotes the necking stress, $\varepsilon_n$ denotes the necking strain, $\varepsilon_y$ denotes the yield strain, $\sigma_{br}$ denotes the breaking stress, $\sigma_y$ denotes the yield stress, d denotes a dart contact diameter, t denotes a thickness of a measurement sample, and b denotes an experimentally determined constant.

In some embodiments, the strain energy may include a third strain energy ($W_{str-hard}$), and where the setting as the feature may include calculating the third strain energy ($W_{str-hard}$) according to the following equation and set the calculated third strain energy ($W_{str-hard}$) as the feature,

$$W_{str-hard} = c\pi t (\varepsilon_{br} - \varepsilon_n) L [d(2\sigma_{br} + \sigma_n) + L(\sigma_{br} + \sigma_n)]$$

where, $\varepsilon_{br}$ denotes the breaking strain, $\varepsilon_n$ denotes the necking strain, $\sigma_{br}$ denotes the breaking stress, $\sigma_n$ denotes the necking stress, L denotes a final stretching propagation length, t denotes a thickness of a measurement sample, and c denotes an experimentally determined constant.

In some embodiments, the strain energy may include a fourth strain energy ($W_{elast}$), and where the setting as the feature may include calculating the fourth strain energy ($W_{elast}$) according to the following equation and set the calculated fourth strain energy ($W_{elast}$) as the feature,

$$W_{elast} = e\pi t\sigma_y(\varepsilon_y)(d + 2L)^2 \ln\left(\frac{D}{d + 2L}\right)$$

where, $\sigma_y$ denotes the yield stress, $\varepsilon_y$ denotes the yield strain, d denotes a dart contact diameter, D denotes a diameter of a fixed disk, L denotes a final stretching propagation length, t denotes a thickness of a measurement sample, and e denotes an experimentally determined constant.

In some embodiments, the generating of the design data for the multilayer film may include combining a predicted value for at least one property of stiffness, strength, strain, tear strength, high-speed dart impact strength, sealing initiation temperature (SIT), haze, moisture permeability, and air permeability with respect to the multilayer film and the predicted value of the dart impact strength, generating the design data based on the combination of the predicted values, when the multilayer film designed based on the combination satisfies the design requirements, and re-designing the multilayer film by changing the combination of the predicted values to another combination, when the multilayer film designed based on the combination does not satisfy the design requirements.

A method for designing the multilayer film according to an embodiment is to be performed by a computing device including one or more processors and one or more memory devices, to design the multilayer film based on a physical property of a multilayer film predicted from only information on a single-layer film, and may include modeling a lamination structure of a multilayer film to be designed, collecting stress-strain data with respect to the single-layer film forming the lamination structure, reading a value pre-stored in a storage space accessible by the computing device, and obtaining a feature setting mode for designating different feature setting manners depending on the read value, calculating a strain energy from a plurality of physical indicators selected from the stress-strain data, depending on the feature setting mode, and setting the strain energy as the feature, selecting at least one among a plurality of supervised learning models capable of a regression analysis as a machine learning model, predicting the dart impact strength of the multilayer film by using the machine learning model learned by taking the feature as an independent variable, and a dart impact strength of the multilayer film as a target variable, and generating design data for the multilayer film, by combining a predicted value for another property and a predicted value of the dart impact strength, so as to satisfy the design requirements of the multilayer film.

In some embodiments, when the feature setting mode may include a first feature setting mode, the setting as the feature may include setting, as the feature, at least some of yield stress, yield strain, necking stress, necking strain, breaking stress, and breaking strain, a strain energy calculated from at least some of the yield stress, the yield strain, the necking stress, the necking strain, the breaking stress, and the breaking strain, and a thickness of the single-layer film.

In some embodiments, the selecting as the machine learning model may include selecting one of a partial least squares (PLS) model, a Lasso regression model, and a random forest model as the machine learning model, and where the machine learning model selected from among the PLS model, the Lasso regression model, and the random forest model is learned to predict the dart impact strength of the multilayer film, by receiving a vector generated from at least some of the yield stress, the yield strain, the necking stress, the necking strain, the breaking stress, and the breaking strain, a vector generated from the strain energy calculated from at least some of the yield stress, the yield strain, the necking stress, the necking strain, the breaking stress, and the breaking strain, and a vector generated from the thickness of the single-layer film.

In some embodiments, when the feature setting mode may include a second feature setting mode, the setting as the feature may include setting, as the feature, yield stress, yield strain, necking stress, necking strain, breaking stress, and breaking strain, a strain energy calculated from the yield stress, the yield strain, the necking stress, the necking strain, the breaking stress, and the breaking strain, and a thickness of the single-layer film.

In some embodiments, the selecting as the machine learning model may include selecting one of a partial least squares (PLS) model, a Lasso regression model, and a random forest model as the machine learning model, and where the machine learning model selected from among the PLS model, the Lasso regression model, and the random forest model is learned to predict the dart impact strength of the multilayer film, by receiving a vector generated from the yield stress, the yield strain, the necking stress, the necking strain, the breaking stress, and the breaking strain, a vector generated from the strain energy calculated from the yield stress, the yield strain, the necking stress, the necking strain, the breaking stress, and the breaking strain, and a vector generated from the thickness of the single-layer film.

In some embodiments, the strain energy may include a first strain energy ($W_{yield}$), and where the setting as the feature may include calculating the first strain energy ($W_{yield}$) according to the following equation and setting the calculated first strain energy ($W_{yield}$) as the feature,

$$W_{yield} = a\pi d^2 t\sigma_y(\varepsilon_y)\left[\left(\frac{\sigma_{br}}{\sigma_y}\right)^2 - 1\right]$$

where, $\sigma_y$ denotes the yield stress, $\varepsilon_y$ denotes the yield strain, $\sigma_{br}$ denotes the breaking stress, d denotes a dart contact diameter, t denotes a thickness of a measurement sample, and a denotes an experimentally determined constant.

In some embodiments, the strain energy may include a second strain energy ($W_{neck}$), and where the setting as the feature may include calculating the second strain energy ($W_{neck}$) according to the following equation and setting the calculated second strain energy ($W_{neck}$) as the feature,

$$W_{neck} = b\pi d^2 t\sigma_n(\varepsilon_n - \varepsilon_y)\left[\left(\frac{\sigma_{br}}{\sigma_y}\right)^2 - 1\right]$$

where, $\sigma_n$ denotes the necking stress, $\varepsilon_n$ denotes the necking strain, $\varepsilon_y$ denotes the yield strain, $\sigma_{br}$ denotes the breaking stress, d denotes a dart contact diameter, $\sigma_y$ denotes the yield stress, t denotes a thickness of a measurement sample, and b denotes an experimentally determined constant.

In some embodiments, the strain energy may include a third strain energy ($W_{str-hard}$), and where the setting as the feature may include calculating the third strain energy ($W_{str-hard}$) according to the following equation and setting the calculated third strain energy ($W_{str-hard}$) as the feature,

$$W_{str-hard} = c\pi t(\varepsilon_{br} - \varepsilon_n)L[d(2\sigma_{br} + \sigma_n) + L(\sigma_{br} + \sigma_n)]$$

where, $\varepsilon_{br}$ denotes the breaking strain, $\varepsilon_n$ denotes the necking strain, $\sigma_{br}$ denotes the breaking stress, $\sigma_n$ denotes the necking stress, L denotes a final stretching propagation length, t denotes a thickness of a measurement sample, and c denotes an experimentally determined constant.

In some embodiments, the strain energy may include a fourth strain energy ($W_{elast}$), and where the setting as the feature may include calculating the fourth strain energy ($W_{elast}$) according to the following equation and setting the calculated fourth strain energy ($W_{elast}$) as the feature,

$$W_{elast} = e\pi t\sigma_y(\varepsilon_y)(d + 2L)^2 \ln\left(\frac{D}{d + 2L}\right)$$

where, $\sigma_y$ denotes the yield stress, $\varepsilon_y$ denotes the yield strain, d denotes a dart contact diameter, D denotes a diameter of a fixed disk, L denotes a final stretching propagation length, t denotes a thickness of a measurement sample, and e denotes an experimentally determined constant.

In some embodiments, the generating of the design data for the multilayer film may include combining a predicted value for at least one property of stiffness, strength, strain, tear strength, high-speed dart impact strength, sealing initiation temperature (SIT), haze, moisture permeability, and air permeability with respect to the multilayer film and the predicted value of the dart impact strength, generating the design data based on the combination of the predicted values, when the multilayer film designed based on the combination satisfies the design requirements, and re-designing the multilayer film by changing the combination of the predicted values to another combination, when the multilayer film designed based on the combination does not satisfy the design requirements.

**[Advantageous Effects]**

According to embodiments, when designing the multilayer film, the multilayer film that can satisfy given design requirements may be designed by predicting the dart impact strength of the multilayer film with only information on the single-layer film constituting the multilayer film, and considering the prediction result on another property together.

**[Description of the Drawings]**

FIG. 1 is a block diagram for explaining an apparatus for designing a multilayer film according to an embodiment.
FIG. 2 is a drawing for explaining stress-strain data with respect to a single-layer film according to an embodiment.
FIG. 3 to FIG. 4 are drawings for explaining dart impact strength measurement environment data according to an embodiment.
FIG. 5 is a flowchart for explaining a method for designing a multilayer film according to an embodiment.
FIG. 6 is a drawing for explaining a computing device according to an embodiment.

**[Mode for Invention]**

Hereinafter, example embodiments of the present disclosure will be described more fully with reference to the accompanying drawings so as to be easily practiced by those skilled in the art to which the present disclosure pertains. As those skilled in the art would realize, the described embodiments may be modified in various different ways, all without departing from the spirit or scope of the present disclosure. In addition, the drawings and description are to be regarded as illustrative in nature and not restrictive, and like reference numerals designate like elements throughout the specification.

Throughout the specification and claims, unless explicitly described to the contrary, the word "comprise" and variations such as "comprises" or "comprising", will be understood to imply the inclusion of stated elements but not the exclusion of any other elements. Terms including an ordinal number such as first, second, etc., may be used to describe various components, but the components are not limited to these terms. The above terms are used solely for the purpose of distinguishing one component from another.

Terms such as "... unit", "... er/or", and "module" used in the specification may mean a unit capable of processing at least one function or operation described in the specification, which may be implemented as hardware or a circuit, software, or a combination of hardware or circuit and software. In addition, at least some components or functions of a design apparatus and method for a multilayer film according to the example embodiments to be described below may be implemented as a program or software, and the program or software may be stored on a computer-readable medium.

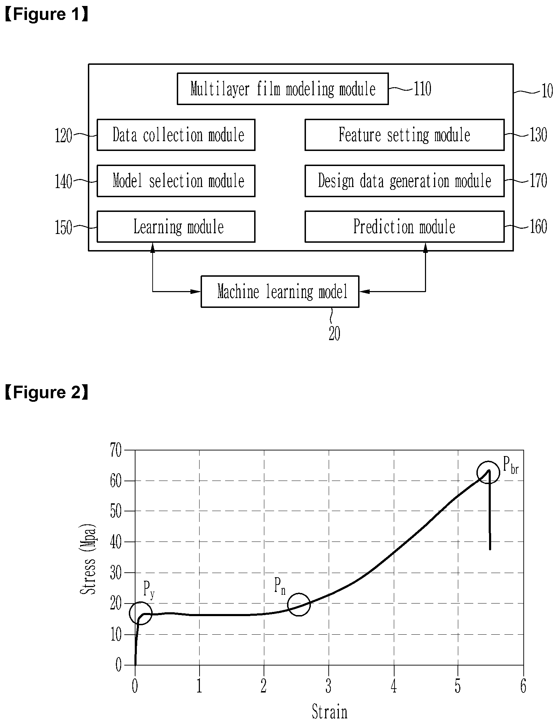

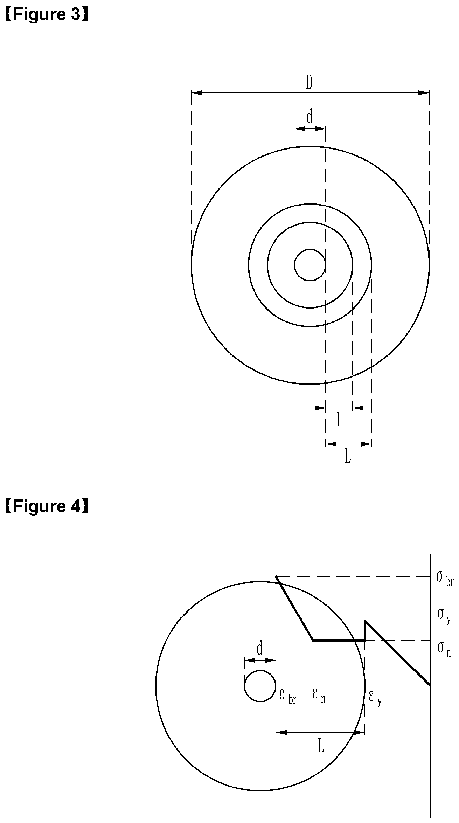

FIG. 1 is a block diagram for explaining an apparatus for designing a multilayer film according to an embodiment. FIG. 2 is a drawing for explaining stress-strain data with respect to a single-layer film according to an embodiment. FIG. 3 to FIG. 4 are drawings for explaining dart impact strength measurement environment data according to an embodiment.

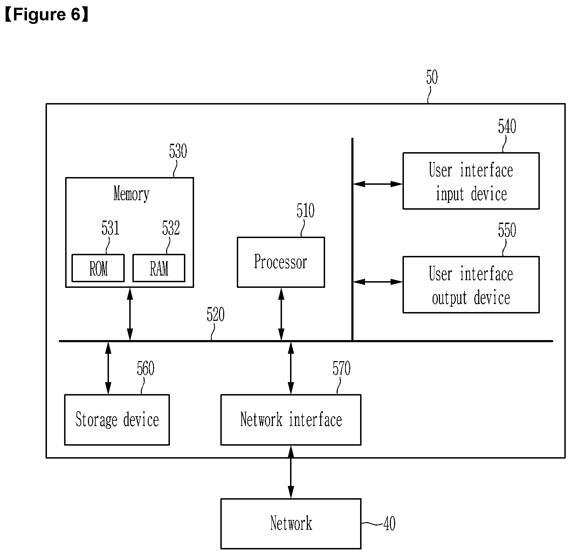

Referring to FIG. 1, an apparatus 10 for designing a multilayer film according to an embodiment may execute a program code or instruction loaded on one or more memory devices through one or more processors. For example, the apparatus 10 for designing a multilayer film may be implemented as a computing device 50 described later with reference to the FIG. 6. In this case, one or more processors may correspond to a processor 510 of the computing device 50, and one or more memory devices may correspond to a memory 530 of the computing device 50. The program code or instruction may be executed by one or more processors, to predict the dart impact strength of the multilayer film with only information on the single-layer film constituting the multilayer film, and design the multilayer film according to given design requirements. In this specification, the term "module" is used to logically separate these functions performed by the program code or instruction.

The apparatus 10 for designing a multilayer film according to an embodiment may predict the dart impact strength of the film without performing a separate dart impact test. The impact strength may mean a strength of physical impact that a material, in this case, a film, can withstand. The impact strength may be used as an indicator to measure the extent of damage occurred to the film, for example, when a weight is dropped from a predetermined height. The apparatus 10 for designing a multilayer film may predict a dart impact strength of the multilayer film through the machine learning based on experimental result data measured in a method other than the dart impact test with respect to the polymer film. To this end, the apparatus 10 for designing a multilayer film may include all or at least some of a multilayer film modeling module 110, a data collection module 120, a feature setting module 130, a model selection module 140, a learning module 150, a prediction module 160, and a design data generation module 170. For example, the learning module 150 may be implemented to be included within the apparatus 10 for designing a multilayer film. Alternatively, for example, the learning module 150 may be implemented outside the apparatus 10 for designing a multilayer film, and the apparatus 10 for designing a multilayer film may be provided with a trained machine learning model from the learning module 150.

The multilayer film modeling module 110 may model a lamination structure of a multilayer film to be designed. The multilayer film may include a plurality of single-layer films. Here, the plurality of single-layer films may include a certain type of single-layer film and a different type of single-layer film, and the unique properties of the single-layer film may vary depending on the type. In addition, a laminating order of the single-layer films within the multilayer film may also vary. The multilayer film modeling module 110 may model the multilayer film, by setting the type of the single-layer film, the laminating order of the single-layer films, or the like, with respect to the multilayer film whose physical properties are to be predicted.

In some embodiments, the multilayer film modeling module 110 may model the multilayer film to include k single-layer films (where k is an integer greater than or equal to 2). For example, the multilayer film modeling module 110 may model a multilayer film in which a first film layer, a second film layer, ..., a k-th film layer are sequentially laminated from the bottom. With respect to each of the k single-layer films forming the multilayer film, features set by the feature setting module 130 to be described later may be applied.

In some embodiments, the multilayer film modeling module 110 may model the multilayer film including the single-layer films of 6 layers. For example, the multilayer film modeling module 110 may model a multilayer film in which the first film layer, the second film layer, a third film layer, a fourth film layer, a fifth film layer, and a sixth film layer are sequentially laminated from the bottom.

The data collection module 120 may collect the stress-strain data with respect to the single-layer film forming a lamination structure modeled by the multilayer film modeling module 110. Here, the stress-strain data may represent mechanical properties of a film (i.e., the single-layer film forming the multilayer film), and referring to FIG. 2 together, may be represented in the form of a stress-strain curve. The stress-strain curve may represent the stress that occurs when a force is applied to a film and how the film is deformed as a result.

In some embodiments, the data collection module 120 may collect the result obtained through a universal testing machine (UTM) experiment on the single-layer films forming the multilayer film as the stress-strain data. An UTM experiment may be designed to perform various mechanical tests on materials, and may measure a load value when a load due to the force load occurs by applying work to a specimen in a certain time, force, and direction. That is, experiments such as compression, tension, and bending can be performed in the manner of applying a force to the specimen and measuring the load value until the specimen deforms. The data collection module 120 may collect the result obtained through the UTM experiment on the specimen of the film, as the stress-strain data.

In some embodiments, the data collection module 120 may collect the dart impact strength data with respect to the single-layer film through a test according to "Method A" of ASTM D1709. Here, the test may be to evaluate the dart impact strength or toughness, or the like of the single-layer film by using a free-falling dart. For more detailed information regarding ASTM D1709, ASTM standard documents can be referred to, and the description thereof will not be included in this specification.

In some embodiments, the data collection module 120 may obtain the stress-strain curve configured based on the result obtained by performing a uniaxial tensile test in a machine direction (MD) and a transverse direction (TD), as data. Here, the MD may be a direction in which the film moves between rollers of a manufacturing machine, in a film manufacturing process, and the TD may be a direction perpendicular to the roller of the manufacturing machine, which is perpendicular to the MD. On the other hand, the uniaxial tensile test may be performed in a manner to measure the resistance of the single-layer film when being stretched in a uniaxial direction. In this case, the data collection module 120 may collect the data with respect to the stress-strain curve through a test according to ASTM D882. For more detailed information regarding ASTM D882, ASTM standard documents can be referred to, and the description thereof will not be included in this specification.

In some embodiments, the stress-strain data may include at least one of yield stress, yield strain, necking stress, necking strain, breaking stress, and breaking strain. Here, the yield stress may represent the stress at the yield point, which is a point where the material exceeds the elastic limit and permanent deformation begins, that is, a point where the material moves from an elastic deformation region to an inelastic deformation region, and the yield strain may represent the strain corresponding to the yield stress. The necking stress may represent the stress at a necking point, which is a point where the cross-section of the material begins to narrow locally, and typically the load capacity of the material may be drastically reduced after the necking point. The necking strain may represent the strain at the necking point. The breaking stress may represent the stress at the point where the material finally breaks, and at the breaking point, the material may no longer withstand the load. The breaking strain may represent the strain corresponding to the breaking stress.

Referring to FIG. 2 together, on the stress-strain curve, an example point $P_y$ corresponding to the yield stress and the yield strain, an example point $P_n$ corresponding to the necking stress and the necking strain, and an example point $P_{br}$ corresponding to the breaking stress and the breaking strain may be determined.

The feature setting module 130 may perform feature setting depending on a plurality of feature setting modes. The plurality of feature setting modes are stored and managed as predetermined values in a storage space accessible by the processor of the apparatus 10 for designing a multilayer film, and the apparatus 10 for designing a multilayer film may read a value pre-stored in that storage space, and determine the feature setting mode for designating different feature setting manners depending on the read value.

The feature setting module 130 may calculate a strain energy from a plurality of physical indicators selected from the stress-strain data, depending on the feature setting mode, and set the strain energy as the feature. In machine learning, the feature may be an input variable used for predicting a target to be predicted, that is, a physical property of the multilayer film, and may mean an individual independent variable of data. The feature may provide information on a target variable or the dependent variable to be predicted within dataset, and have a significant influence on the performance of a machine learning model 20. The learning module 150 may perform the learning of the machine learning model 20 by using the feature. At this time, the feature may constitute a part of data input to the machine learning model 20, and the machine learning model 20 may learn the pattern based on those data and perform predictions. In particular, feature engineering, which selects or transforms features, may be an important process for improving the performance of the machine learning model 20. That is, selecting appropriate features or appropriately transforming features can have a significant influence on the high performance of the machine learning model 20.

In some embodiments, when the feature setting mode includes a first feature setting mode, the feature setting module 130 may set at least some of the yield stress, the yield strain, the necking stress, the necking strain, the breaking stress, and the breaking strain, a strain energy calculated from at least some of the yield stress, the yield strain, the necking stress, the necking strain, the breaking stress, and the breaking strain, and a thickness of the single-layer film, as the feature. That is, the feature setting module 130 may set at least a part of the stress-strain data collected by the data collection module 120, a strain energy calculated from at least a part of the stress-strain data collected by the data collection module 120, and the thickness of the single-layer film, as the features. When the multilayer film including the k single-layer films is modeled by the multilayer film modeling module 110, the learning module 150 may apply the features including only a part of the stress-strain data collected by the data collection module 120 with respect to the first film layer among the k single-layer films, the strain energy calculated from at least a part of the stress-strain data collected by the data collection module 120, and the thickness of the single-layer film. In addition, the learning module 150 may apply the features including only a part of the stress-strain data collected by the data collection module 120 with respect to the second film layer among the k single-layer films, the strain energy calculated from at least a part of the stress-strain data collected by the data collection module 120, and the thickness of the single-layer film. In addition, the learning module 150 may perform the learning of the machine learning model 20 by applying the features including only a part of the stress-strain data collected by the data collection module 120 with respect to the k-th film layer, the strain energy calculated from at least a part of the stress-strain data collected by the data collection module 120, and the thickness of the single-layer film. At this time, the learning may be performed by applying the same type of feature to all of the k single-layer films, and alternatively, the learning may be performed so that a different type of feature from other film layers may be applied to one film layer among the k single-layer films. This embodiment may be adopted in an environment where computing resources for performing learning and prediction of the machine learning model 20 are limited.

In some embodiments, when the feature setting mode includes a second feature setting mode, the feature setting module 130 may set the yield stress, the yield strain, the necking stress, the necking strain, the breaking stress, and the breaking strain, a strain energy calculated from the yield stress, the yield strain, the necking stress, the necking strain, the breaking stress, and the breaking strain, and the thickness of the single-layer film, as the features. That is, the feature setting module 130 may set all of the stress-strain data collected by the data collection module 120, a strain energy calculated from the stress-strain data collected by the data collection module 120, and the thickness of the single-layer film, as the features. When the multilayer film including the k single-layer films is modeled by the multilayer film modeling module 110, the learning module 150 may apply the features including all of the stress-strain data collected by the data collection module 120 with respect to the first film layer among the k single-layer films, the strain energy calculated from the stress-strain data collected by the data collection module 120, and the thickness of the single-layer film. In addition, the learning module 150 may apply the features including all of the stress-strain data collected by the data collection module 120 with respect to the second film layer among the k single-layer films, the strain energy calculated from the stress-strain data collected by the data collection module 120, and the thickness of the single-layer film. In addition, the learning module 150 may perform the learning of the machine learning model 20 by applying the features including all of the stress-strain data collected by the data collection module 120 with respect to the k-th film layer, the strain energy calculated from the stress-strain data collected by the data collection module 120, and the thickness of the single-layer film. At this time, the learning may be performed by applying the same type of feature to all of the k single-layer films, and alternatively, the learning may be performed so that a different type of feature from other film layers may be applied to one film layer among the k single-layer films. This embodiment may be adopted in an environment where computing resources for performing learning and prediction of the machine learning model 20 are sufficient.

In some embodiments, the stress-strain data may include first stress-strain data and second stress-strain data. The first stress-strain data may represent mechanical properties of the single-layer film in a first direction, and the second stress-strain data may represent mechanical properties of the single-layer film in a second direction perpendicular to the first direction. Specifically, the first direction may be the MD, and the second direction may be the TD. The feature setting module 130 may set the plurality of physical indicators selected from at least one of the first stress-strain data and the second stress-strain data, as the features.

In some embodiments, when the feature setting mode includes a third feature setting mode, the feature setting module 130 may set at least some of the yield stress, the yield strain, the necking stress, the necking strain, the breaking stress, and the breaking strain in the first direction, the strain energy calculated from at least some of the yield stress, the yield strain, the necking stress, the necking strain, the breaking stress, and the breaking strain in the first direction, at least some of the yield stress, the yield strain, the necking stress, the necking strain, the breaking stress, and the breaking strain in the second direction, the strain energy calculated from at least some of the yield stress, the yield strain, the necking stress, the necking strain, the breaking stress, and the breaking strain in the second direction, and the thickness of the single-layer film, as the features. That is, the feature setting module 130 may set at least a part of the first stress-strain data collected by the data collection module 120, a strain energy calculated from at least a part of the first stress-strain data collected by the data collection module 120, at least a part of the second stress-strain data collected by the data collection module 120, a strain energy calculated from at least a part of the second stress-strain data collected by the data collection module 120, and the thickness of the single-layer film, as the features. When the multilayer film including the k single-layer films is modeled by the multilayer film modeling module 110, the learning module 150 may apply the features including only a part of the first stress-strain data collected by the data collection module 120 with respect to the first film layer among the k single-layer films, the strain energy calculated from at least a part of the first stress-strain data collected by the data collection module 120, only a part of the second stress-strain data collected by the data collection module 120, the strain energy calculated from at least a part of the second stress-strain data collected by the data collection module 120, and the thickness of the single-layer film. In addition, the learning module 150 may apply the features including only a part of the first stress-strain data collected by the data collection module 120 with respect to the second film layer among the k single-layer films, the strain energy calculated from at least a part of the first stress-strain data collected by the data collection module 120, only a part of the second stress-strain data collected by the data collection module 120, the strain energy calculated from at least a part of the second stress-strain data collected by the data collection module 120, and the thickness of the single-layer film. In addition, the learning module 150 may perform the learning of the machine learning model 20, by applying the feature including only a part of the first stress-strain data collected by the data collection module 120 with respect to the k-th film layer, the strain energy calculated from at least a part of the first stress-strain data collected by the data collection module 120, only a part of the second stress-strain data collected by the data collection module 120, the strain energy calculated from at least a part of the second stress-strain data collected by the data collection module 120, and the thickness of the single-layer film. At this time, the learning may be performed by applying the same type of feature to all of the k single-layer films, and alternatively, the learning may be performed so that a different type of feature from other film layers may be applied to one film layer among the k single-layer films. This embodiment may be adopted in an environment where computing resources for performing learning and prediction of the machine learning model 20 are limited.

In some embodiments, when the feature setting mode includes a fourth feature setting mode, the feature setting module 130 may set the yield stress, the yield strain, the necking stress, the necking strain, the breaking stress, and the breaking strain in the first direction, the strain energy calculated from the yield stress, the yield strain, the necking stress, the necking strain, the breaking stress, and the breaking strain in the first direction, the yield stress, the yield strain, the necking stress, the necking strain, the breaking stress, and the breaking strain in the second direction, the strain energy calculated from the yield stress, the yield strain, the necking stress, the necking strain, the breaking stress, and the breaking strain in the second direction, and the thickness of the single-layer film, as the features. That is, the feature setting module 130 may set all of the first stress-strain data collected by the data collection module 120, a strain energy calculated from the first stress-strain data collected by the data collection module 120, all of the second stress-strain data collected by the data collection module 120, a strain energy calculated from the second stress-strain data collected by the data collection module 120, and the thickness of the single-layer film, as the features. When the multilayer film including the k single-layer films is modeled by the multilayer film modeling module 110, the learning module 150 may apply the features including all of the first stress-strain data collected by the data collection module 120 with respect to the first film layer among the k single-layer films, the strain energy calculated from the first stress-strain data collected by the data collection module 120, all of the second stress-strain data collected by the data collection module 120, the strain energy calculated from the second stress-strain data collected by the data collection module 120, and the thickness of the single-layer film. In addition, the learning module 150 may apply the features including all of the first stress-strain data collected by the data collection module 120 with respect to the second film layer among the k single-layer films, the strain energy calculated from the first stress-strain data collected by the data collection module 120, all of the second stress-strain data collected by the data collection module 120, the strain energy calculated from the second stress-strain data collected by the data collection module 120, and the thickness of the single-layer film. In addition, the learning module 150 may perform the learning of the machine learning model 20, by applying the features including all of the first stress-strain data collected by the data collection module 120 with respect to the k-th film layer, the strain energy calculated from the first stress-strain data collected by the data collection module 120, all of the second stress-strain data collected by the data collection module 120, the strain energy calculated from the second stress-strain data collected by the data collection module 120, and the thickness of the single-layer film. At this time, the learning may be performed by applying the same type of feature to all of the k single-layer films, and alternatively, the learning may be performed so that a different type of feature from other film layers may be applied to one film layer among the k single-layer films. This embodiment may be adopted in an environment where computing resources for performing learning and prediction of the machine learning model 20 are sufficient.

For example, when the multilayer film modeling module 110 models a multilayer film including the single-layer films of 6 layers, 21 features of all of indicators of the yield stress, the yield strain, the necking stress, the necking strain, the breaking stress, and the breaking strain in the first direction, four indicators of the strain energy calculated from the yield stress, the yield strain, the necking stress, the necking strain, the breaking stress, and the breaking strain in the first direction, all of indicators of the yield stress, the yield strain, the necking stress, the necking strain, the breaking stress, and the breaking strain in the second direction, four indicators of the strain energy calculated from the yield stress, the yield strain, the necking stress, the necking strain, the breaking stress, and the breaking strain in the second direction, and the thickness of the single-layer film may be applied to one film layer, and since there exist 6 layers in total, 126 features in total may be applied.

In some embodiments, the feature setting module 130 may calculate a first strain energy ($W_{yield}$) from the stress-strain data and set it as a feature. Specifically, the feature setting module 130 may calculate the first strain energy ($W_{yield}$) according to the following Equation 1.

### Equation 1

$$W_{yield} = a\pi d^2 t \sigma_y(\varepsilon_y)\left[\left(\frac{\sigma_{br}}{\sigma_y}\right)^2 - 1\right]$$

Here, $\sigma_y$ may denote the yield stress among the stress-strain data, $\varepsilon_y$ may denote the yield strain among the stress-strain data, and $\sigma_{br}$ of the stress-strain data may denote the breaking stress. These variables may correspond to experimental result data measured in a method other than the dart impact test as described above.

On the other hand, in order to calculate a strain energy from the stress-strain data, variables related to the environmental setting of a dart impact test according to a certain predetermined standard may be additionally used for the film whose dart impact strength is to be predicted. Here, the standard may represent the American Society for Testing and Materials (ASTM) standard, and may represent any other standard that is customized in consideration of the learning performance of the machine learning model 20. Specifically, as variables related to the environmental setting of the dart impact test, d may denote a dart contact diameter, t may denote a thickness of a measurement sample, and a may denote an experimentally determined constant in consideration of the experiment environment or product test environment. Referring to FIG. 3 together, the junction diameter d may mean a diameter of a circular area bonded to an additional film when a weight is dropped from a predetermined height. The weight include a fixed disk having a diameter D, the area denoted by I is an area over which stretching propagates, and the area denoted by L represents an area (or length) over which stretching ultimately propagates. Variables (d, D, I, L, t, or the like) related to the environmental setting of separate dart impact tests may be appropriately adjusted in order to improve the accuracy of the dart impact strength to be ultimately predicted.

In some embodiments, the feature setting module 130 may calculate a second strain energy ($W_{neck}$) from the stress-strain data and set it as a feature. Specifically, the feature setting module 130 may calculate the second strain energy ($W_{neck}$) according to the following Equation 2.

### Equation 2

$$W_{neck} = b\pi d^2 t \sigma_n(\varepsilon_n - \varepsilon_y)\left[\left(\frac{\sigma_{br}}{\sigma_y}\right)^2 - 1\right]$$

Here, $\sigma_n$ may denote the necking stress among the stress-strain data, $\varepsilon_n$ may denote the necking strain among the stress-strain data, $\varepsilon_y$ may denote the yield strain among the stress-strain data, $\sigma_{br}$ may denote the breaking stress among the stress-strain data, $\sigma_y$ may denote the yield stress among the stress-strain data, and these variables may correspond to experimental result data measured in a method other than the dart impact test as described above.

On the other hand, as a variable related to environmental setting of the dart impact test, d may be the dart contact diameter. t may denote the thickness of the measurement sample, and b may denote an experimentally determined constant in consideration of the experiment environment or product test environment.

In some embodiments, the feature setting module 130 may calculate a third strain energy ($W_{str-hard}$) from the stress-strain data and set it as a feature. Specifically, the feature setting module 130 may calculate the third strain energy ($W_{str-hard}$) according to the following Equation 3.

### Equation 3

$$W_{str-hard} = c\pi t(\varepsilon_{br} - \varepsilon_n)L[d(2\sigma_{br} + \sigma_n) + L(\sigma_{br} + \sigma_n)]$$

Here, $\varepsilon_{br}$ may denote the breaking strain among the stress-strain data, $\varepsilon_n$ may denote the necking strain among the stress-strain data, $\sigma_{br}$ may denote the breaking stress among the stress-strain data, and $\sigma_n$ may denote the necking stress among the stress-strain data. These variables may correspond to experimental result data measured in a method other than the dart impact test as described above.

On the other hand, as a variable related to environmental setting of the dart impact test, L may denote final stretching propagation length, t may denote the thickness of the measurement sample, and c may denote an experimentally determined constant in consideration of the experiment environment or product test environment.

In some embodiments, the feature setting module 130 may calculate a fourth strain energy ($W_{elast}$) from the stress-strain data and set it as a feature. Specifically, the feature setting module 130 may calculate the fourth strain energy

($W_{elast}$) according to the following Equation 4.

## Equation 4

$$W_{elast} = e\pi t\sigma_y(\varepsilon_y)(d + 2L)^2 \ln\left(\frac{D}{d + 2L}\right)$$

Here, $\sigma_y$ may denote the yield stress among the stress-strain data, and $\varepsilon_y$ may denote the yield strain among the stress-strain data. These variables may correspond to experimental result data measured in a method other than the dart impact test as described above.

On the other hand, as a variable related to environmental setting of the dart impact test, d may denote the dart contact diameter, D may denote a diameter of the fixed disk, L may denote final stretching propagation length, t may denote the thickness of the measurement sample, and e may denote a predetermined constant.

In some embodiments, a final stretching propagation length L may be calculated from experimental result data measured in a method other than the dart impact test. Specifically, the final stretching propagation length L may be calculated according to the following Equation 5.

## Equation 5

$$L = \frac{(\sigma_{br} - \sigma_y)d}{2\sigma_y}$$

Here, $\sigma_{br}$ may denote the breaking stress among the stress-strain data, $\sigma_y$ may denote the yield stress among the stress-strain data, d may be the dart contact diameter.

In some embodiments, the feature setting module 130 may set the first strain energy ($W_{yield}$), the second strain energy ($W_{neck}$), the third strain energy ($W_{str-hard}$), and the fourth strain energy ($W_{elast}$), as features, altogether. The learning module 150 may perform the learning of the machine learning model 20 based on the feature set in this manner. This embodiment may be adopted in an environment where computing resources for performing learning and prediction of the machine learning model 20 are sufficient.

Alternatively, in some other embodiments, the feature setting module 130 may set at least some of the first strain energy ($W_{yield}$), the second strain energy ($W_{neck}$), the third strain energy ($W_{str-hard}$), and the fourth strain energy ($W_{elast}$), as the feature. The learning module 150 may perform the learning of the machine learning model 20 based on the feature set in this manner. This embodiment may be adopted in an environment where computing resources for performing learning and prediction of the machine learning model 20 are limited.

In some embodiments, the feature setting module 130 may additionally set the yield stress, the yield strain, the necking stress, the necking strain, the breaking stress, and the breaking strain, as the features. That is, the feature setting module 130 may also set the yield stress, the yield strain, the necking stress, the necking strain, the breaking stress, and the breaking strain selected from the stress-strain data, together with the strain energy calculated from the stress-strain data, as features.

In some embodiments, the feature setting module 130 may additionally set the thickness of the single-layer film forming the multilayer film, as the feature. That is, the feature setting module 130 may also set the thickness of the single-layer film, together with the strain energy calculated from the stress-strain data, as features.

In some embodiments, the feature setting module 130 may set at least one or all of the first strain energy ($W_{yield}$), the second strain energy ($W_{neck}$), the third strain energy ($W_{str-hard}$), and the fourth strain energy ($W_{elast}$) in the first direction and at least one or all of the first strain energy ($W_{yield}$), the second strain energy ($W_{neck}$), the third strain energy ($W_{str-hard}$), and the fourth strain energy ($W_{elast}$) in the second direction, as features. That is, the feature setting module 130 may set at least some or all of strain energies calculated from the first stress-strain data collected by the data collection module 120, at least some or all of strain energies calculated from the second stress-strain data, selectively together with the yield stress, the yield strain, the necking stress, the necking strain, the breaking stress, and the breaking strain, and selectively together with the thickness of the single-layer film, as features.

The model selection module 140 may select the machine learning model to be used for predicting a physical property, that is, the dart impact strength of the multilayer film. Specifically, the model selection module 140 may select at least one among a plurality of supervised learning models capable of a regression analysis as machine learning model for the dart impact strength of the multilayer film prediction. The supervised learning is a method of training a model by using labeled training data, and in the supervised learning, the model can learn the relationship between input variables and the target variable, and through this, can predict a value of the target variable with respect to new input data. In some embodiments, the model selection module 140 may select the supervised learning model capable of regression analysis, from among linear regression, polynomial regression, ridge regression, Lasso regression, logistic regression, decision trees, random forests, gradient boosting, and neural networks. The PLS model may be used for modeling the relationship between the various independent variables and the various dependent variables (or the target variables), and may find the independent variables determined as being important for prediction of the dependent variables. In the linear regression, the model may learn the linear relationship between the input variable and the target variable, and in the polynomial regression, the model may learn complex relationships by expanding the linear regression, to include higher-order terms of the input variable. The ridge regression and the Lasso regression may prevent overfitting by adding regularization to linear regression, and adjust the complexity of model, and the logistic regression is a classification algorithm for solving binary classification problems, but it can be used for the regression analysis because it predicts the result as a probability. Decision trees, random forests, and gradient boosting learns complex nonlinear relationships, thereby possibly being used also for regression problems as well as classification problems, neural networks can solve various types of regression problems, and deep learning in particular can be very effective for learning complex nonlinear relationships. Of course, the supervised learning model capable of regression analysis that can be selected by the model selection module 140 is not limited what have been listed in the above, and another type of model that is not listed can be selected in consideration of specific implementation purpose, implementation environment, required performance, or the like.

The learning module 150 may train the machine learning model 20, taking the feature set by the feature setting module 130 as the independent variable, and the dart impact strength of the multilayer film as the target variable. Accordingly, the machine learning model 20 may learn the relationship between the features and the target variables to be trained as a model that can predict the physical properties of the multilayer film with respect to new data, that is, the physical property data with respect to the single-layer film. In particular, the learning module 150 may use different learning methods depending on the model selected by the model selection module 140.

The prediction module 160 may predict the dart impact strength of the multilayer film by using the machine learning model 20 trained by the learning module 150. That is, the prediction module 160 may transfer the new data, that is, the physical property data with respect to the single-layer film, to the machine learning model 20, and obtain the prediction result with respect to the dart impact strength of the multilayer film.

In more detail, the model selection module 140 may perform model selection depending on a plurality of model selection modes. The plurality of model selection modes are stored and managed as predetermined values in the storage space accessible by the processor of the apparatus 10 for designing a multilayer film, and the apparatus 10 for designing a multilayer film may read a value pre-stored in that storage space, and determine the model selection mode for designating different model selection methods depending on according to the read value.

In more detail, the model selection module 140 may perform model selection according to the plurality of model selection modes. The plurality of model selection modes are stored and managed as predetermined values in the storage space accessible by the processor of the apparatus 10 for designing a multilayer film, and the apparatus 10 for designing a multilayer film may read a value pre-stored in that storage space, and determine the model selection mode for designating different model selection methods depending on according to the read value.

In some embodiments, when the model selection mode is a first model selection mode, the model selection module 140 may select the PLS model as the machine learning model to be used for predicting the dart impact strength of the multilayer film. As described above, the feature set by the feature setting module 130 depending on the feature setting mode may be combined in the form of, for example, a single vector, and used as an input to the PLS model. The impact strength of the multilayer film is set as the target variable, and the learning module 150 may train the PLS model in a direction that maximizes the correlation between the input feature and the target variable. At this time, the learning module 150 may optimize the "n_components" parameter so that the PLS model properly explain the data, and the optimal "n_components" value may be determined through the intersection verification. Through the PLS model learned in this way, the dart impact strength of the multilayer film may be predicted by inputting features with respect to a new single-layer film.

In some other embodiments, when the model selection mode is a second model selection mode, the model selection module 140 may select a Lasso regression model as the machine learning model to be used for predicting the dart impact strength of the multilayer film. As described above, the feature set by the feature setting module 130 depending on the feature setting mode may be combined in the form of, for example, a single vector, and used as an input to the Lasso regression model. The impact strength of the multilayer film is set as the target variable, and the learning module 150 may train the Lasso regression model to induce the model to select only important features through L1 regularization (regularization that adds the sum of absolute values of regression coefficients to the object function as penalty). At this time, the learning module 150 may optimize an "alpha" parameter for adjusting the regulation strength during model learning, and the optimal "alpha" value may be determined through the intersection verification. Through the Lasso regression model learned in this way, the dart impact strength of the multilayer film may be predicted by inputting features with respect to a new single-layer film.

Still another in some embodiments, when the model selection mode is a third model selection mode, the model selection module 140 may select a random forest model as the machine learning model to be used for predicting the dart impact strength of the multilayer film. As described above, the feature set by the feature setting module 130 depending on the feature setting mode may be combined in the form of, for example, a single vector, and used as an input to the random forest model. The impact strength of the multilayer film is set as the target variable, and the learning module 150 may perform training by setting "n_estimators", "max_depth", "min_samples_split", or the like as hyperparameters of the random forest model. Through the random forest model learned in this way, the dart impact strength of the multilayer film may be predicted by inputting features with respect to a new single-layer film.

In some embodiments, the machine learning model selected from among the PLS model, the Lasso regression model, and the random forest model may be configured to receive a vector generated from the at least some of the yield stress, the yield strain, the necking stress, the necking strain, the breaking stress, and the breaking strain, a vector generated from the strain energy calculated from at least some of the yield stress, the yield strain, the necking stress, the necking strain, the breaking stress, and the breaking strain, and a vector generated from the thickness of the single-layer film, and learned to predict the dart impact strength of the multilayer film.

In some other embodiments, the machine learning model selected from among the PLS model, the Lasso regression model, and the random forest model may be configured to receive a vector generated from the yield stress, the yield strain, the necking stress, the necking strain, the breaking stress, and the breaking strain, a vector generated from the strain energy calculated from the yield stress, the yield strain, the necking stress, the necking strain, the breaking stress, and the breaking strain, and a vector generated from the thickness of the single-layer film, and learned to predict the dart impact strength of the multilayer film.

The design data generation module 170 may primarily combine a predicted value for at least one property of stiffness, strength, strain, tear strength, high-speed dart impact strength, sealing initiation temperature, haze, moisture permeability, and air permeability with respect to the multilayer film and a predicted value of the dart impact strength. The design requirements with respect to the multilayer film to be designed may include requirements for ensuring mechanical properties, thermal properties, optical properties, barrier properties, chemical properties, or the like, to secure desired performance. When the multilayer film designed based on a primary combination of the predicted values satisfies the design requirements, the design data generation module 170 may generate design data based on the primary combination. In this case, the design data may include specific predicted values.

Alternatively, when the multilayer film designed based on the primary combination of the predicted values does not satisfy the design requirements, the design data generation module 170 may re-design the multilayer film by changing the primary combination to another combination. That is, the design data generation module 170 may secondarily combine the predicted value for at least one property of stiffness, strength, strain, tear strength, high-speed dart impact strength, sealing initiation temperature, haze, moisture permeability, and air permeability with respect to the multilayer film and the predicted value of the dart impact strength, to have a different combination from the primary combination. At this time, the predicted value of the dart impact strength in the primary combination and the predicted value of the dart impact strength in a secondary combination may be different values.

When the multilayer film designed based on the secondary combination of the predicted values satisfies the design requirements, the design data generation module 170 may generate design data based on the secondary combination. Alternatively, when the multilayer film designed based on the secondary combination of the predicted values does not satisfy the design requirements either, the design data generation module 170 may repeat the process of re-designing the multilayer film by changing the secondary combination to still another combination.

According to the present embodiment, without directly performing the dart impact test on the film in order to measure the dart impact strength, by performing the learning and prediction by taking the strain energy calculated from the stress-strain data measured in a method other than the dart impact test as the feature, with respect to the single-layer film forming the multilayer film, the dart impact strength of the multilayer film can be predicted with a high accuracy. In addition, by differentiating the number of indicators selected as features to prioritize performance and the number of indicators selected as features to save computing resources, flexible design is possible according to implementation purpose and environment of an apparatus for designing a multilayer film. Furthermore, in consideration of the prediction result related to other properties of the multilayer film together with the prediction result of the dart impact strength, the multilayer film that can satisfy given design requirements can be designed.

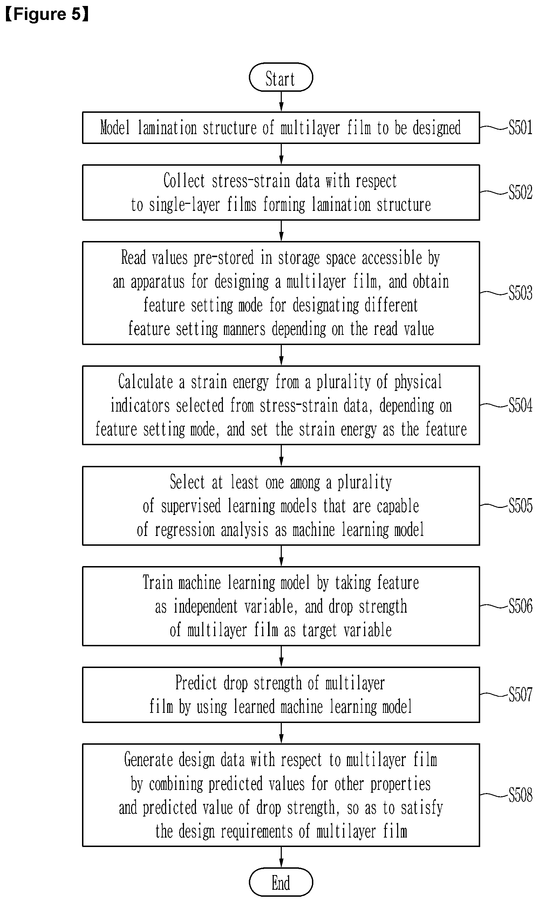

FIG. 5 is a flowchart for explaining a method for designing a multilayer film of a multi-layered material according to an embodiment.

Referring to FIG. 5, a method for designing a multilayer film according to an embodiment may include a step S501 of modeling a lamination structure of the multilayer film to be designed, a step S502 of collecting the stress-strain data with respect to the single-layer film forming the lamination structure, a step S503 of reading a value pre-stored in a storage space accessible by an apparatus for designing a multilayer film and obtaining the feature setting mode for designating different feature setting manners depending on the read value, a step S504 of calculating a strain energy from the plurality of physical indicators selected from the stress-strain data, depending on the feature setting mode, and setting the strain energy as the feature, a step S505 of selecting at least one among the plurality of supervised learning models capable of regression analysis as the machine learning model, a step S506 of training the machine learning model, taking the feature as the independent variable, and the dart impact strength of the multilayer film as the target variable, a step S507 of predicting the dart impact strength of the multilayer film by using the learned machine learning model, and a step S508 of generating the design data with respect to the multilayer film by combining predicted values for other properties and the predicted value of the dart impact strength, so as to satisfy the design requirements of the multilayer film.

Further details of a method for designing a multilayer film may be referred to the characteristic configurations disclosed with reference to the description of embodiments described in this specification, and the redundant description is not included herein.

FIG. 6 is a drawing for explaining a computing device according to an embodiment.

Referring to FIG. 6, an apparatus and method for designing a multilayer film according to embodiments may be implemented by using the computing device 50. The computing device 50 may be implemented in various forms of electronic devices, servers, or similar devices, and its function may be implemented through a combination of software and hardware.

The computing device 50 may include at least one of the processor 510, a memory 530, a user interface input device 540, a user interface output device 550, and a storage device 560 that communicate through a bus 520. The computing device 50 may also include a network interface 570 electrically connected to a network 40. The network interface 570 may transmit or receive signal with other entities through the network 40.

The processor 510 may be implemented as various types of computational devices, for example, a micro controller unit (MCU), an application processor (AP), a central processing unit (CPU), a graphics processing unit (GPU), a neural processing unit (NPU), quantum processing unit (QPU), or the like. The processor 510 is a semiconductor device that execute instructions stored in the memory 530 or the storage device 560, and may play a core role of the system. The program codes and data stored in the memory 530 or the storage device 560 may command the processor 510 to perform a specific work, thereby enabling the overall operation of the system. Through this, the processor 510 may be configured to implement various functions and methods described with reference to FIG. 1 to FIG. 5.

The memory 530 and the storage device 560 may include various types of volatile or non-volatile storage media, for storing and accessing data of the system. For example, the memory 530 may include a read-only memory (ROM) 531 and a random-access memory (RAM) 532. In some embodiments, the memory 530 may be embedded inside the processor 510, and in this case, the data transmission speed between the memory 530 and the processor 510 may be very fast. In some other embodiments, the memory 530 may be located outside the processor 510, and in this case, the memory 530 may be connected to the processor 510 through various data buses or interfaces. This connection may be made through already known various means, for example, a Peripheral Component Interconnect Express (PCIe) interface for high-rate data transmission or a memory controller.

In some embodiments, at least some components and features of an apparatus and method for designing a multilayer film according to embodiments may be implemented as a program or software executed by the computing device 50, and the program or software may be stored in a computer-readable medium. Specifically, a computer-readable medium according to an embodiment may record a program for causing a computer including the processor 510 executing program or instruction stored in the memory 530 or the storage device 560 to execute steps included in an apparatus and method for designing a multilayer film according to embodiments.

In some embodiments, at least some components and features of an apparatus and method for designing a multilayer film according to embodiments may be implemented using hardware or circuitry of the computing device 50, or may be implemented as separate hardware or circuitry that can be electrically connected to the computing device 50.

According to embodiments, without directly performing the dart impact test on the film in order to measure the dart impact strength, by performing the learning and prediction by taking the strain energy calculated from the stress-strain data measured in a method other than the dart impact test as the feature, with respect to the single-layer film forming the multilayer film, the dart impact strength of the multilayer film can be predicted with a high accuracy. In addition, by differentiating the number of indicators selected as features to prioritize performance and the number of indicators selected as features to save computing resources, flexible design is possible according to implementation purpose and environment of an apparatus and method for designing a multilayer film. Furthermore, in consideration of the prediction result related to other properties of the multilayer film together with the prediction result of the dart impact strength, the multilayer film that can satisfy given design requirements can be designed.

Although embodiments of the present disclosure have been described in detail hereinabove, the scope of the present disclosure is not limited thereto, but may include several modifications and alterations made by those skilled in the art to which the present disclosure pertains using a basic concept of the present disclosure as defined in the claims.

**Claims**

1.　An apparatus for designing a multilayer film, the apparatus designing a multilayer film based on a physical property of the multilayer film predicted from only information on a single-layer film, by executing a program code loaded on one or more memory devices through one or more processors,
wherein the program code is configured, when executed, to perform:

modeling a lamination structure of a multilayer film to be designed;
collecting stress-strain data with respect to the single-layer film forming the lamination structure;
reading a value pre-stored in a storage space accessible by an apparatus for designing a multilayer film, and obtaining a feature setting mode for designating different feature setting manners depending on the read value;
calculating a strain energy from a plurality of physical indicators selected from the stress-strain data, depending on the feature setting mode, and setting the strain energy as a feature;
selecting at least one among a plurality of supervised learning models capable of a regression analysis as a machine learning model;
predicting a dart impact strength of the multilayer film by using the machine learning model learned by taking the feature as an independent variable, and the dart impact strength of the multilayer film as a target variable; and
generating design data for the multilayer film, by combining a predicted value for another property and a predicted value of the dart impact strength, so as to satisfy design requirements of the multilayer film.

2. The apparatus of claim 1, wherein, when the feature setting mode comprises a first feature setting mode, the setting as the feature comprises setting, as the feature:

at least some of yield stress, yield strain, necking stress, necking strain, breaking stress, and breaking strain;
a strain energy calculated from at least some of the yield stress, the yield strain, the necking stress, the necking strain, the breaking stress, and the breaking strain; and
a thickness of the single-layer film.

3. The apparatus of claim 2, wherein

the selecting as the machine learning model comprises selecting one of a partial least squares (PLS) model, a Lasso regression model, and a random forest model as the machine learning model, and
wherein the machine learning model selected from among the PLS model, the Lasso regression model, and the random forest model is learned to predict the dart impact strength of the multilayer film, by receiving:

a vector generated from at least some of the yield stress, the yield strain, the necking stress, the necking strain, the breaking stress, and the breaking strain;
a vector generated from the strain energy calculated from at least some of the yield stress, the yield strain, the necking stress, the necking strain, the breaking stress, and the breaking strain; and
a vector generated from the thickness of the single-layer film.

4. The apparatus of claim 1, wherein, when the feature setting mode comprises a second feature setting mode, the setting as the feature comprises setting, as the feature:

yield stress, yield strain, necking stress, necking strain, breaking stress, and breaking strain;
a strain energy calculated from the yield stress, the yield strain, the necking stress, the necking strain, the breaking stress, and the breaking strain; and
a thickness of the single-layer film.

5. The apparatus of claim 4, wherein:

the selecting as the machine learning model comprises selecting one of a partial least squares (PLS) model, a Lasso regression model, and a random forest model as the machine learning model, and
wherein the machine learning model selected from among the PLS model, the Lasso regression model, and the random forest model are learned to predict the dart impact strength of the multilayer film, by receiving:

a vector generated from the yield stress, the yield strain, the necking stress, the necking strain, the breaking stress, and the breaking strain;
a vector generated from the strain energy calculated from the yield stress, the yield strain, the necking stress, the necking strain, the breaking stress, and the breaking strain; and
a vector generated from the thickness of the single-layer film.

6. The apparatus of claim 2, wherein the strain energy comprises a first strain energy ($W_{yield}$), and wherein the setting as the feature comprises:

calculating the first strain energy ($W_{yield}$) according to the following equation and setting the calculated first strain energy ($W_{yield}$) as the feature,

$$W_{yield} = a\pi d^2 t\sigma_y(\varepsilon_y)\left[\left(\frac{\sigma_{br}}{\sigma_y}\right)^2 - 1\right]$$

where, $\sigma_y$ denotes the yield stress, $\varepsilon_y$ denotes the yield strain, $\sigma_{br}$ denotes the breaking stress, d denotes a dart contact diameter, t denotes a thickness of a measurement sample, and a denotes an experimentally determined constant.

7. The apparatus of claim 2, wherein the strain energy comprises a second strain energy ($W_{neck}$), and wherein setting as the feature comprises:

calculating the second strain energy ($W_{neck}$) according to the following equation and set the calculated second strain energy ($W_{neck}$) as the feature,

$$W_{neck} = b\pi d^2 t\sigma_n(\varepsilon_n - \varepsilon_y)\left[\left(\frac{\sigma_{br}}{\sigma_y}\right)^2 - 1\right]$$

where, $\sigma_n$ denotes the necking stress, $\varepsilon_n$ denotes the necking strain, $\varepsilon_y$ denotes the yield strain, $\sigma_{br}$ denotes the breaking stress, $\sigma_y$ denotes the yield stress, d denotes a dart contact diameter, t denotes a thickness of a measurement sample, and b denotes an experimentally determined constant.

8. The apparatus of claim 2, wherein the strain energy comprises a third strain energy ($W_{str-hard}$), and wherein the setting as the feature comprises:

calculating the third strain energy ($W_{str-hard}$) according to the following equation and set the calculated third strain energy ($W_{str-hard}$) as the feature,

$$W_{str-hard} = c\pi t(\varepsilon_{br} - \varepsilon_n)L[d(2\sigma_{br} + \sigma_n) + L(\sigma_{br} + \sigma_n)]$$

where, $\varepsilon_{br}$ denotes the breaking strain, $\varepsilon_n$ denotes the necking strain, $\sigma_{br}$ denotes the breaking stress, $\sigma_n$ denotes the necking stress, L denotes a final stretching propagation length, t denotes a thickness of a measurement sample, and c denotes an experimentally determined constant.

9. The apparatus of claim 2, wherein the strain energy comprises a fourth strain energy ($W_{elast}$), and wherein the setting as the feature comprises:

calculating the fourth strain energy ($W_{elast}$) according to the following equation and set the calculated fourth strain energy ($W_{elast}$) as the feature,

$$W_{elast} = e\pi t\sigma_y(\varepsilon_y)(d + 2L)^2 \ln\left(\frac{D}{d + 2L}\right)$$

where, $\sigma_y$ denotes the yield stress, $\varepsilon_y$ denotes the yield strain, d denotes a dart contact diameter, D denotes a diameter of a fixed disk, L denotes a final stretching propagation length, t denotes a thickness of a measurement sample, and e denotes an experimentally determined constant.

10. The apparatus of claim 1, wherein the generating of the design data for the multilayer film comprises:

combining a predicted value for at least one property of stiffness, strength, strain, tear strength, high-speed dart impact strength, sealing initiation temperature (SIT), haze, moisture permeability, and air permeability with respect to the multilayer film and the predicted value of the dart impact strength;

generating the design data based on the combination of the predicted values, when the multilayer film designed based on the combination satisfies the design requirements; and

re-designing the multilayer film by changing the combination of the predicted values to another combination, when the multilayer film designed based on the combination does not satisfy the design requirements.

11. A method for designing a multilayer film, the method being performed by a computing device comprising one or more processors and one or more memory devices, to design a multilayer film based on a physical property of the multilayer film predicted from only information on a single-layer film, the method comprising:

modeling a lamination structure of a multilayer film to be designed;

collecting stress-strain data with respect to the single-layer film forming the lamination structure;

reading a value pre-stored in a storage space accessible by the computing device, and obtaining a feature setting mode for designating different feature setting manners depending on the read value;

calculating a strain energy from a plurality of physical indicators selected from the stress-strain data, depending on the feature setting mode, and setting the strain energy as a feature;

selecting at least one among a plurality of supervised learning models capable of a regression analysis as a machine learning model;

predicting a dart impact strength of the multilayer film by using the machine learning model learned by taking the feature as an independent variable, and the dart impact strength of the multilayer film as a target variable; and

generating design data for the multilayer film, by combining a predicted value for another property and a predicted value of the dart impact strength, so as to satisfy design requirements of the multilayer film.

12. The method of claim 11, wherein, when the feature setting mode comprises a first feature setting mode, the setting as the feature comprises setting, as the feature:

at least some of yield stress, yield strain, necking stress, necking strain, breaking stress, and breaking strain;

a strain energy calculated from at least some of the yield stress, the yield strain, the necking stress, the necking strain, the breaking stress, and the breaking strain; and

a thickness of the single-layer film.

13. The method of claim 12, wherein the selecting as the machine learning model comprises selecting one of a partial least squares (PLS) model, a Lasso regression model, and a random forest model as the machine learning model, and wherein the machine learning model selected from among the PLS model, the Lasso regression model, and the random forest model is learned to predict the dart impact strength of the multilayer film, by receiving:

a vector generated from at least some of the yield stress, the yield strain, the necking stress, the necking strain, the breaking stress, and the breaking strain;

a vector generated from the strain energy calculated from at least some of the yield stress, the yield strain, the necking stress, the necking strain, the breaking stress, and the breaking strain; and

a vector generated from the thickness of the single-layer film.

14. The method of claim 11, wherein, when the feature setting mode comprises a second feature setting mode, the setting as the feature comprises setting, as the feature:

yield stress, yield strain, necking stress, necking strain, breaking stress, and breaking strain;

a strain energy calculated from the yield stress, the yield strain, the necking stress, the necking strain, the breaking stress, and the breaking strain; and

a thickness of the single-layer film.

15. The method of claim 14, wherein the selecting as the machine learning model comprises selecting one of a partial least squares (PLS) model, a Lasso regression model, and a random forest model as the machine learning model, and wherein the machine learning model selected from among the PLS model, the Lasso regression model, and the random forest model is learned to predict the dart impact strength of the multilayer film, by receiving:

a vector generated from the yield stress, the yield strain, the necking stress, the necking strain, the breaking stress, and the breaking strain;

a vector generated from the strain energy calculated from the yield stress, the yield strain, the necking stress, the necking strain, the breaking stress, and the breaking strain; and
a vector generated from the thickness of the single-layer film.

16. The method of claim 12, wherein the strain energy comprises a first strain energy ($W_{yield}$), and wherein the setting as the feature comprises:

calculating the first strain energy ($W_{yield}$) according to the following equation and setting the calculated first strain energy ($W_{yield}$) as the feature,

$$W_{yield} = a\pi d^2 t \sigma_y (\varepsilon_y) \left[ \left( \frac{\sigma_{br}}{\sigma_y} \right)^2 - 1 \right]$$

where, $\sigma_y$ denotes the yield stress, $\varepsilon_y$ denotes the yield strain, $\sigma_{br}$ denotes the breaking stress, d denotes a dart contact diameter, t denotes a thickness of a measurement sample, and a denotes an experimentally determined constant.

17. The method of claim 12, wherein the strain energy comprises a second strain energy ($W_{neck}$), and wherein the setting as the feature comprises:

calculating the second strain energy ($W_{neck}$) according to the following equation and setting the calculated second strain energy ($W_{neck}$) as the feature,

$$W_{neck} = b\pi d^2 t \sigma_n (\varepsilon_n - \varepsilon_y) \left[ \left( \frac{\sigma_{br}}{\sigma_y} \right)^2 - 1 \right]$$

where, $\sigma_n$ denotes the necking stress, $\varepsilon_n$ denotes the necking strain, $\varepsilon_y$ denotes the yield strain, $\sigma_{br}$ denotes the breaking stress, d denotes a dart contact diameter, $\sigma_y$ denotes the yield stress, t denotes a thickness of a measurement sample, and b denotes an experimentally determined constant.

18. The method of claim 12, wherein the strain energy comprises a third strain energy ($W_{str-hard}$), and wherein the setting as the feature comprises:

calculating the third strain energy ($W_{str-hard}$) according to the following equation and setting the calculated third strain energy ($W_{str-hard}$) as the feature,

$$W_{str-hard} = c\pi t (\varepsilon_{br} - \varepsilon_n) L [d(2\sigma_{br} + \sigma_n) + L(\sigma_{br} + \sigma_n)]$$

where, $\varepsilon_{br}$ denotes the breaking strain, $\varepsilon_n$ denotes the necking strain, $\sigma_{br}$ denotes the breaking stress, $\sigma_n$ denotes the necking stress, L denotes a final stretching propagation length, t denotes a thickness of a measurement sample, and c denotes an experimentally determined constant.

19. The method of claim 12, wherein the strain energy comprises a fourth strain energy ($W_{elast}$), and wherein the setting as the feature comprises:

calculating the fourth strain energy ($W_{elast}$) according to the following equation and setting the calculated fourth strain energy ($W_{elast}$) as the feature,

$$W_{elast} = e\pi t \sigma_y (\varepsilon_y) (d + 2L)^2 \ln \left( \frac{D}{d + 2L} \right)$$

where, $\sigma_y$ denotes the yield stress, $\varepsilon_y$ denotes the yield strain, d denotes a dart contact diameter, D denotes a diameter of a fixed disk, L denotes a final stretching propagation length, t denotes a thickness of a measurement sample, and e denotes an experimentally determined constant.

**20.** The method of claim 11, wherein the generating of the design data for the multilayer film comprises:

combining a predicted value for at least one property of stiffness, strength, strain, tear strength, high-speed dart impact strength, sealing initiation temperature (SIT), haze, moisture permeability, and air permeability with respect to the multilayer film and the predicted value of the dart impact strength;
generating the design data based on the combination of the predicted values, when the multilayer film designed based on the combination satisfies the design requirements; and
re-designing the multilayer film by changing the combination of the predicted values to another combination, when the multilayer film designed based on the combination does not satisfy the design requirements.

【Figure 1】

【Figure 2】

【Figure 3】

【Figure 4】

【Figure 5】

```
                        ( Start )
                            │
                            ▼
┌──────────────────────────────────────────────────────┐
│ Model lamination structure of multilayer film to be designed │───S501
└──────────────────────────────────────────────────────┘
                            │
                            ▼
┌──────────────────────────────────────────────────────┐
│           Collect stress-strain data with respect       │
│      to single-layer films forming lamination structure  │───S502
└──────────────────────────────────────────────────────┘
                            │
                            ▼
┌──────────────────────────────────────────────────────┐
│        Read values pre-stored in storage space accessible by  │
│     an apparatus for designing a multilayer film, and obtain   │
│           feature setting mode for designating different       │───S503
│        feature setting manners depending on the read value     │
└──────────────────────────────────────────────────────┘
                            │
                            ▼
┌──────────────────────────────────────────────────────┐
│       Calculate a strain energy from a plurality of physical   │
│      indicators selected from stress-strain data, depending on │───S504
│   feature setting mode, and set the strain energy as the feature │
└──────────────────────────────────────────────────────┘
                            │
                            ▼
┌──────────────────────────────────────────────────────┐
│              Select at least one among a plurality            │
│          of supervised learning models that are capable       │───S505
│          of regression analysis as machine learning model     │
└──────────────────────────────────────────────────────┘
                            │
                            ▼
┌──────────────────────────────────────────────────────┐
│         Train machine learning model by taking feature        │
│          as independent variable, and drop strength           │───S506
│              of multilayer film as target variable            │
└──────────────────────────────────────────────────────┘
                            │
                            ▼
┌──────────────────────────────────────────────────────┐
│              Predict drop strength of multilayer              │
│          film by using learned machine learning model         │───S507
└──────────────────────────────────────────────────────┘
                            │
                            ▼
┌──────────────────────────────────────────────────────┐
│      Generate design data with respect to multilayer film     │
│       by combining predicted values for other properties      │───S508
│       and predicted value of drop strength, so as to satisfy  │
│             the design requirements of multilayer film        │
└──────────────────────────────────────────────────────┘
                            │
                            ▼
                        ( End )
```

【Figure 6】

## INTERNATIONAL SEARCH REPORT

| | |
|---|---|
| International application No. | |
| | **PCT/KR2024/014627** |

| | |
|---|---|
| **A. CLASSIFICATION OF SUBJECT MATTER** | |
| **G01N 3/303**(2006.01)i; **G01N 33/44**(2006.01)i; **G06N 20/00**(2019.01)i | |
| According to International Patent Classification (IPC) or to both national classification and IPC | |
| **B. FIELDS SEARCHED** | |
| Minimum documentation searched (classification system followed by classification symbols) | |
| G01N 3/303(2006.01); B29C 70/02(2006.01); G01N 19/08(2006.01); G01N 3/00(2006.01); G01N 3/30(2006.01); G01N 33/44(2006.01); G06F 17/50(2006.01); G06N 3/08(2006.01); G16C 20/30(2019.01); G16C 60/00(2019.01) | |
| Documentation searched other than minimum documentation to the extent that such documents are included in the fields searched | |
| Korean utility models and applications for utility models: IPC as above<br>Japanese utility models and applications for utility models: IPC as above | |
| Electronic data base consulted during the international search (name of data base and, where practicable, search terms used) | |
| eKOMPASS (KIPO internal) & keywords: 다층 필름(multilayer film), 응력 변형(stress-strain), 낙추강도(drop strength), 기계학습(machine learning), 회귀분석(Regression Analysis), 설계(design), 항복응력(yield stress), PLS(Partial Least Squares) | |

**C. DOCUMENTS CONSIDERED TO BE RELEVANT**

| Category* | Citation of document, with indication, where appropriate, of the relevant passages | Relevant to claim No. |
|---|---|---|
| A | KR 10-2023-0055484 A (LG CHEM, LTD.) 26 April 2023 (2023-04-26)<br>See paragraphs [0132]-[0161], claims 1 and 6 and figures 1-3. | 1-20 |
| A | US 2015-0170022 A1 (KING FAHD UNIVERSITY OF PETROLEUM AND MINERALS) 18 June 2015 (2015-06-18)<br>See paragraphs [0518]-[0554], claim 1 and figures 35 and 36. | 1-20 |
| A | KR 10-2017-0054564 A (SABIC GLOBAL TECHNOLOGIES B.V.) 17 May 2017 (2017-05-17)<br>See paragraphs [0026]-[0092], claim 1 and figures 3 and 4. | 1-20 |
| A | KR 10-2023-0096707 A (LG CHEM, LTD.) 30 June 2023 (2023-06-30)<br>See claims 1-5. | 1-20 |

☑ Further documents are listed in the continuation of Box C.   ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br><br>**02 January 2025** | Date of mailing of the international search report<br><br>**03 January 2025** |
| Name and mailing address of the ISA/KR<br><br>**Korean Intellectual Property Office<br>Government Complex-Daejeon Building 4, 189 Cheongsa-ro, Seo-gu, Daejeon 35208** | Authorized officer<br><br> |
| Facsimile No. **+82-42-481-8578** | Telephone No. |

Form PCT/ISA/210 (second sheet) (July 2022)

## INTERNATIONAL SEARCH REPORT

International application No.

**PCT/KR2024/014627**

### C. DOCUMENTS CONSIDERED TO BE RELEVANT

| Category* | Citation of document, with indication, where appropriate, of the relevant passages | Relevant to claim No. |
|---|---|---|
| A | US 2017-0322145 A1 (U.S.A., AS REPRESENTED BY THE ADMINISTRATOR OF THE NATIONAL AERONAUTICS AND SPACE ADMINISTRATION) 09 November 2017 (2017-11-09)<br>See claim 1 and figure 1. | 1-20 |

## INTERNATIONAL SEARCH REPORT
### Information on patent family members

| | International application No. |
|---|---|
| | **PCT/KR2024/014627** |

| Patent document cited in search report | | | Publication date (day/month/year) | Patent family member(s) | | | Publication date (day/month/year) |
|---|---|---|---|---|---|---|---|
| KR | 10-2023-0055484 | A | 26 April 2023 | CN | 116249886 | A | 09 June 2023 |
| | | | | CN | 116368365 | A | 30 June 2023 |
| | | | | CN | 116368366 | A | 30 June 2023 |
| | | | | EP | 4202402 | A1 | 28 June 2023 |
| | | | | EP | 4209773 | A1 | 12 July 2023 |
| | | | | EP | 4209774 | A1 | 12 July 2023 |
| | | | | JP | 2023-542990 | A | 12 October 2023 |
| | | | | JP | 2023-546423 | A | 02 November 2023 |
| | | | | JP | 2023-550883 | A | 06 December 2023 |
| | | | | JP | 7540856 | B2 | 27 August 2024 |
| | | | | JP | 7558522 | B2 | 01 October 2024 |
| | | | | KR | 10-2023-0033577 | A | 08 March 2023 |
| | | | | KR | 10-2023-0033578 | A | 08 March 2023 |
| | | | | KR | 10-2023-0056575 | A | 27 April 2023 |
| | | | | US | 2023-0334195 | A1 | 19 October 2023 |
| | | | | US | 2023-0394193 | A1 | 07 December 2023 |
| | | | | US | 2024-0290438 | A1 | 29 August 2024 |
| | | | | WO | 2023-027444 | A1 | 02 March 2023 |
| | | | | WO | 2023-027445 | A1 | 02 March 2023 |
| | | | | WO | 2023-068520 | A1 | 27 April 2023 |
| US | 2015-0170022 | A1 | 18 June 2015 | US | 9274036 | B2 | 01 March 2016 |
| KR | 10-2017-0054564 | A | 17 May 2017 | CN | 106796617 | A | 31 May 2017 |
| | | | | CN | 106796617 | B | 25 September 2018 |
| | | | | EP | 3180193 | A1 | 21 June 2017 |
| | | | | JP | 2018-156689 | A | 04 October 2018 |
| | | | | JP | 2018-503884 | A | 08 February 2018 |
| | | | | JP | 6356339 | B2 | 11 July 2018 |
| | | | | KR | 10-1780173 | B1 | 19 September 2017 |
| | | | | US | 2017-0371980 | A1 | 28 December 2017 |
| | | | | WO | 2017-027598 | A1 | 16 February 2017 |
| KR | 10-2023-0096707 | A | 30 June 2023 | | None | | |
| US | 2017-0322145 | A1 | 09 November 2017 | US | 10036700 | B2 | 31 July 2018 |
| | | | | WO | 2016-076965 | A1 | 19 May 2016 |

Form PCT/ISA/210 (patent family annex) (July 2022)

**EP 4 764 452 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230129784
- KR 1020240130233